Europäisches Patentamt

European Patent Office

Office européen des brevets

(19)

(11) Veröffentlichungsnummer: **0 167 070**
**A2**

(12) **EUROPÄISCHE PATENTANMELDUNG**

(21) Anmeldenummer: **85107672.9**

(22) Anmeldetag: **21.06.85**

(51) Int. Cl.⁴: **C 07 H 17/00**

(30) Priorität: **30.06.84 DE 3424183**
**02.02.85 DE 3503620**

(43) Veröffentlichungstag der Anmeldung:
**08.01.86 Patentblatt 86/2**

(84) Benannte Vertragsstaaten:
**AT BE CH DE FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Ihiem, Joachim, Prof. Dr.**
**Asbeckweg 31**
**D-4400 Münster(DE)**

(72) Erfinder: **Deger, Hans-Matthias, Dr.**
**Am Rheingauer Weg 8**
**D-6238 Hofheim am Taunus(DE)**

(72) Erfinder: **Kolar, Cenek, Dr.**
**Deutschhausstrasse 20**
**D-3550 Marburg 1(DE)**

(72) Erfinder: **Kreuzer, Matthias**
**Nottulner Landweg 21**
**D-4400 Münster(DE)**

(54) **Verfahren zur Herstellung von 1-Azidoaldosen.**

(57) 1-Azidoaldosen erhält man durch Umsetzung von 1-Fluor-Aldosen mit Metallaziden in polaren, wassermischbaren, vorzugsweise wasserhaltigen Lösemitteln. Die Hydroxygruppen reagieren hierbei nicht, so daß deren Schutz entbehrlich ist bzw. sie in derivatisierter Form vorliegen können.

EP 0 167 070 A2

Verfahren zur Herstellung von 1-Azidoaldosen

Sowohl die klassische also auch die moderne synthetische Kohlenhydratchemie ist auf die breite Verwendung aufwendiger Schutzgruppentechniken angewiesen, die teils zur Durchführung selektiver Reaktionen am polyfunktionellen Zuckermolekül, teils jedoch zur Stabilisierung von im ungeschützten Zustand labilen Verbindungen unvermeidbar sind. Durch den hohen Aufwand an Chemikalien, Zeit und Arbeit wird die breite technische Anwendung von Kohlenhydratderivaten stark eingeschränkt.

So können z.B. ß-1-Azidoaldosen, die Zwischenprodukte zur Synthese von beispielsweise 1,6-Anhydrozuckern (F. Micheel et al., Chem. Ber. 88,475 (1955) und 88, 479 (1955)) und Triazolyl-N-glycosiden (F. Micheel et al., Chem. Ber. 90, 1595 (1957)), nur über eine vierstufige Synthese aus Aldosen gewonnen werden (R.U. Lemieux in Meth. Carbohydr. Chem. II, S. 221, Academic Press 1963, und A. Bertho, Chem. Ber. 63, 841 (1930)). Die Einführung der Azidgruppe erfolgt durch Umsetzung von Tetraacetyl-α-glycosyl-bromid mit Natriumazid unter Wasserauschluß zu Tetraacetyl-ß-glycosylazid, aus dem durch Abspaltung der Acetylgruppen die freie ß-Azidoaldose hergestellt werden kann (A. Bertho, a.a.O.).

Überraschenderweise wurde nun gefunden, daß 1-Azidoaldosen direkt, also ohne Verwendung von mit Schutzgruppen versehenen Zwischenstufen, hergestellt werden können, indem man ungeschützte 1-Fluoraldosen mit Metallaziden in Gegenwart eines polaren wassermischbaren organischen Lösemittels umsetzt. Hierbei werden die Azidoaldosen unter Konfigurationsumkehr (Inversion) oder Konfigurationserhalt (Retention) gebildet. Die Hydroxygruppen reagieren hierbei nicht, so daß deren Schutz entbehrlich ist bzw. sie in derivatisierter Form vorliegen können.

Die Zugabe von Wasser zur Reaktionsmischung erhöht im allgemeinen die Reaktionsgeschwindigkeit erheblich.

Als Ausgangsmaterialien eignen sich die α- oder ß-1-Fluor-aldosen aus der Reihe der Pentosen, wie α- oder ß-1-Fluor-xylose, -arabinose oder -ribose, aus der Reihe der Hexosen beispielsweise α- oder ß-1-Fluorglucose, -galaktose, -mannose, -gulose oder -altrose, aus der Reihe der Di-saccharide beispielsweise die α- oder ß-1-Fluor-maltose, -cellobiose, -lactose, -isomaltose oder -gentiobiose, aber auch Derivate dieser Zucker wie beispielsweise α- oder ß-1-Fluor-N-acetyl-2-amino-2-desoxy-glucose.

Als Metallazide eignen sich alle wasserlöslichen Azide, vorzugsweise die Erdalkaliazide wie Calciumazid, und die Alkaliazide wie Natrium- und Kaliumazid. Die Erdalkaliazide können als solche, aber auch in statu nascendi, z.B. aus Natriumazid und Calciumchlorid, umgesetzt werden. Da es sich um eine stöchiometrische Umsetzung handelt, wird das Metallazid wenigstens in der stöchiometrischen Menge, meistens jedoch in einem den praktischen Verhältnissen entsprechenden Überschuß verwendet. Die bevorzugte Menge liegt zwischen einem 50 %igen Überschuß und dem Dreifachen der stöchiometrisch benötigten Menge.

Als wassermischbare organische Lösemittel eignen sich polare protische und aprotische Lösemittel, beispielsweise niedere Alkanole wie Methanol, Äthanol, Propanol und Isopropanol, Acetonitril, Amide wie Formamid, Dimethylformamid oder N-Methylpyrrolidon sowie Äther wie Mono- oder Dimethoxyäthan, Monoäthoxyäthan oder Tetrahydrofuran.

Die Reaktion wird vorteilhaft bei Temperaturen ab 0°C, bei Raumtemperatur oder erhöhter Temperatur durchgeführt,

vorzugsweise bei 40 - 120°C, insbesondere bei 60 - 100°C, vor allem, wenn Alkaliazide verwendet werden. Die Umsetzung mit Erdalkaliaziden, insbesondere Calciumazid, läßt sich mit Vorteil schon bei den niedrigeren Temperaturen ab 0°C durchführen, vorzugsweise von Raumtemperatur bis 60°C. Beim Einsatz von niedrig siedenden organischen Lösemitteln kann unter Druck gearbeitet werden, also beispielsweise im geschlossenen Gefäß; vorteilhaft wählt man jedoch als Obergrenze den Siedepunkt des Reaktionssystems, wobei man also in der Praxis zweckmäßig unter Rückfluß arbeitet. Die Abtrennung der gebildeten Salze kann in üblicher Weise vorgenommen werden. Vorteilhaft trennt man sie mit Hilfe eines Ionenaustauschers oder über einem Molekularsieb in Wasser oder Gemischen davon mit den vorgenannten wassermischbaren organischen Lösemitteln ab.

In den folgenden Beispielen werden vorteilhafte Ausgestaltungen der Erfindung näher erläutert.

Beispiele
1) Herstellung von ß-D-Glucopyranosylazid = Verbindung 1

9,1 g α-D-Glucosylfluorid wurden in einem Gemisch aus 50 ml Methanol und 10 ml Wasser mit 3,57 g Natriumazid 48 Stunden unter Rückfluß erhitzt. Das Methanol wurde anschließend verdampft, der Rückstand in 50 ml Wasser aufgenommen und mit einem Ionenaustauscher entsalzt. Das Wasser wurde unter dem Vakuum der Wasserstrahlpumpe abgedampft und der ölige Rückstand durch Destillation mit Äthanol entwässert. Der so erhaltene Sirup wurde bei 50°C im Hochvakuum vom restlichen Äthanol befreit. Man erhielt 9,7 g eines leicht gelb gefärbten Sirups, der nach gaschromatographischer Analyse zu 92 % aus ß-1-Azidoglucose bestand.

Verfuhr man analog, jedoch unter Ausschluß von Wasser, so konnte die ß-1-Azidoglucose (nach gleicher Reaktionsdauer) nur in geringen Mengen nachgewiesen werden.

2) Herstellung von ß-D-Glucopyranosylazid = Verbindung 1

9,1 g ß-D-Glucosylfluorid wurden in einem Gemisch aus 50 ml Acetonitril und 10 ml Wasser mit 3,57 g Natriumazid 24 Stunden bei 70°C gerührt. Nach Aufarbeitung wie in Beispiel 1 erhielt man 9,0 g eines Sirups, der zu 78 % aus ß-D-Azidoglucose und zu 6 % aus D-Glucose bestand.

3) Herstellung von ß-D-Galactopyranosylazid = Verbindung 2

9,1 g α-D-Galactosylfuorid wurden in einem Gemisch aus 50 ml Methanol und 10 ml Wasser mit 3,57 g Natriumazid 24 Stunden unter Rückfluß erhitzt. Nach Aufarbeitung wie in Beispiel 1 erhielt man 9,6 g eines Sirups, der zu 94 % aus ß-D-Azidogalactose bestand. Durch Acetylierung und Umkristallisation aus Isopropanol wurden 13,2 g (71 % d. Th.) Tetraacetyl-ß-D-azidogalactose vom Schmelzpunkt 101 - 103°C erhalten.

4) Herstellung von ß-Cellobiosylazid= Verbindung 3

6,9 α-Cellobiosylfluorid wurden in einem Gemisch aus 70 ml Äthanol und 15 ml Wasser mit 1,42 g Natriumazid 72 Stunden bei 60°C gerührt. Nach Aufarbeitung wie in Beispiel 1 erhielt man 9,6 g eines Sirups, der zu 85 % aus ß-Azidocellobiose bestand.

5) Herstellung von α-D-Mannopyranosylazid= Verbindung 4

12 g α-D-Mannosylfluorid wurden in 100 ml Acetonitril mit 4,7 g Natriumazid 80 Stunden auf Rückflußtemperatur erhitzt. Nach Aufarbeitung wie in Beispiel 1 erhielt man 10,3 g eines Sirups, der zu 92 % aus α-Azidomannose bestand.

6)  Herstellung der Verbindung 1

94,9 g (0,855 Mol) Calciumchlorid und 129,7 g (1,995 Mol) Natriumazid wurden in 800 ml 95 %igem wäßrigen Methanol suspendiert. Nach 30 min Rühren bei Raumtemperatur wurde die Suspension, die nun das Calciumazid enthielt, in situ mit 55,4 g (0,285 Mol) α-D-Glucopyranosylfluorid, gelöst in 200 ml Methanol, versetzt. Die Suspension wurde 15 Std. bei Raumtemperatur gerührt, wobei der Reaktionsverlauf dünnschichtchromatographisch (Laufmittel: Chloroform/ Methanol 3:1) verfolgt wurde. Nach der Beendigung der Reaktion wurde die Suspension abfiltriert und der Rückstand wurde mit Äthanol nachgewaschen. Die vereinigten Filtrate wurden im Vakuum der Wasserstrahlpumpe eingedampft. Der resultierende Sirup wurde in Äthanol gelöst, die Lösung wurde über Molekularsieb 4 A filtriert und zum Sirup im Vakuum der Wasserstrahlpumpe eingeengt. Ausbeute: 54 g (92.3 %); IR: 2115 cm$^{-1}$ (-N$_3$)

Das ß-Glucopyranosylazid wurde als 2,3,4,6-Tetra-O-acetyl-ß-D-glucopyranosylazid NMR-spektroskopisch sowie polarimetrisch ($[\alpha]^{20}$ = -32° c= 1 in Chloroform; Literaturwert $[\alpha]^{20}$ = -33° c= 2,48 in Chloroform gemäß A. Bertho und D. Aures, Liebigs Ann. Chem. 592, 54 (1955)) nachgewiesen.

7)  Wie im Beispiel 6 beschrieben, wurden die folgenden 1-Azidoaldosen hergestellt und IR- und NMR-spektroskopisch als deren Peracetate charakterisiert:
ß-D-Galatopyranosylazid            (Verbindung 2)
ß-Cellobiosylazid                  (Verbindung 3)
α-D-Mannopyranosylazid             (Verbindung 4)

PATENTANSPRÜCHE:

1. Verfahren zur Herstellung von 1-Azidoaldosen, dadurch gekennzeichnet, daß man eine 1-Fluor-aldose mit einem Metallazid in Gegenwart eines polaren wassermischbaren Lösemittels umsetzt.

2. Verfahren nach Anspruch 1, dadurch gekennzeichnet, daß die Umsetzung im Bereich von 0°C bis zum Siedepunkt des Reaktionssystems durchgeführt wird.

3. Verfahren nach Anspruch 1, dadurch gekennzeichnet, daß daß die Umsetzung bei 40 - 120°C, vorzugsweise bei 60 - 100°C durchgeführt wird.

4. Verfahren nach einem oder mehreren der Ansprüche 1 bis 3, dadurch gekennzeichnet, daß das polare wassermischbare Lösemittel ein niederes Alkanol, Acetonitril, ein Amid oder ein Äther ist.

5. Verfahren nach einem oder mehreren der vorhergehenden Ansprüche, dadurch gekennzeichnet, daß die Reaktionsmischung Wasser enthält.

6. Verfahren nach einem oder mehreren der vorhergehenden Ansprüche, dadurch gekennzeichnet, daß aus dem Reaktionsgemisch die Salze mit Hilfe eines Ionenaustauschers abgetrennt werden.

7. Verfahren nach einem oder mehreren der vorhergehenden Ansprüche, dadurch gekennzeichnet, daß als Metallazid ein Erdalkalimetall- oder Alkalimetall-azid, vorzugsweise Natrium- oder Kaliumazid eingesetzt wird.

8. Verfahren nach Anspruch 7, dadurch gekennzeichnet, daß als Metallazid ein Erdalkaliazid, insbesondere Calciumazid eingesetzt wird, wobei vorzugsweise von Raumtemperatur bis 60°C gearbeitet wird.

9. Verfahren nach einem oder mehreren der vorhergehenden Ansprüche, dadurch gekennzeichnet, daß das Metallazid wenigstens in der stöchiometrischen Menge, vorzugsweise in einer Menge zwischen einem 50 %igen Überschuß und dem Dreifachen der stöchiometrisch benötigten Menge angewandt wird und daß das Erdalkaliazid vorzugsweise in statu nascendi umgesetzt wird.

10. Verfahren nach einem oder mehreren der Ansprüche 1 - 5 und 7 bis 9, dadurch gekennzeichnet, daß die Salze aus dem Reaktionsgemisch über einem Molekularsieb in Wasser oder Gemischen davon mit wenigstens einem wassermischbaren organischen Lösemittel abgetrennt werden.